# EUROPEAN PATENT APPLICATION

(11) **EP 4 461 723 A1**
(43) Date of publication of application: **13.11.2024**
(21) Application number: 23737005.1
(22) Date of filing: 04.01.2023
(51) Int. Cl.: C07C 209/28, C07C 211/55, C09K 15/16, C08L 7/00

(54) **N-(3,5,5-TRIMETHYLCYCLOHEXYL)-N'-PHENYL P-PHENYLENEDIAMINE, AND SYNTHESIS METHOD THEREFOR**

(30) Priority: 04.01.2022 CN 202210004337
(71) Applicant: Sennics Co., Ltd., China (Shanghai) Pilot Free Trade Zone 200120 (CN)
(72) Inventor: MIAO, Zhengan, Shanghai 200120 (CN); LI, Shiwu, Shanghai 200120 (CN); GAO, Yang, Shanghai 200120 (CN); QIU, Lingling, Shanghai 200120 (CN); ZHANG, Pingting, Shanghai 200120 (CN)
(74) Representative: V.O.
(86) International application number: PCT/CN2023/070250
(87) International publication number: WO 2023/131140

(57) **Abstract**

Disclosed in the present invention are a novel p-phenylenediamine rubber anti-aging agent containing a cycloalkyl structure: N-(3,5,5-trimethylcyclohexyl)-N'-phenyl p-phenylenediamine, and a synthesis method therefor. The present rubber anti-aging agent can increase the vulcanization speed of a rubber material, shorten scorching time, improve heat-resistant oxygen aging performance of the rubber, and at the same time, keep good original physical properties, flexing resistance and dynamic fatigue resistance.

## Description

### FIELD OF THE INVENTION

The present invention belongs to the field of p-phenylenediamine antidegradants, specifically relates to N-(3,5,5-trimethylcyclohexyl)-N'-phenyl p-phenylenediamine, and synthesis method therefor.

### BACKGROUND

P-phenylenediamine antidegradants are a class of high-performance rubber antidegradants that have a wide range of applications in tires, tapes, and other industrial rubber products.

Antidegradant 6PPD, also known as N-(1,3-dimethylbutyl)-N'-phenyl p-phenylenediamine, is a widely used p-phenylenediamine antidegradant in rubber products such as tires. It can provide good original physical properties, flexing resistance and dynamic fatigue resistance to rubber products such as tires. However, the vulcanization speed, scorching time, and thermal oxidative aging resistance of a rubber material need to be improved.

Therefore, there is a need in this field for an antidegradant that can increase the vulcanization speed, shorten scorching time, and improve thermal oxidative aging resistance of the rubber material.

### SUMMARY

To overcome the above problems, the present invention provides a novel p-phenylenediamine rubber antidegradant containing a cycloalkyl structure-N-(3,5,5-trimethylcyclohexyl)-N'-phenyl p-phenylenediamine, and a synthesis method therefor. The present rubber anti-aging agent can increase the vulcanization speed of a rubber material, shorten scorching time, improve thermal oxidative aging resistance of the rubber, and at the same time, keep good original physical properties, flexing resistance and dynamic fatigue resistance.

Specifically, one aspect of the present invention provides a compound (N-(3,5,5-trimethylcyclohexyl)-N'-phenyl p-phenylenediamine) with the structure shown in the following formula:

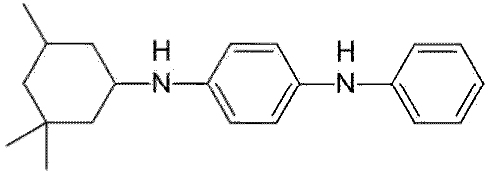

Another aspect of the present invention provides a method for preparing N-(3,5,5-trimethylcyclohexyl)-N'-phenyl p-phenylenediamine, wherein the method comprises reacting a compound A, 4-aminodiphenylamine, and hydrogen in a condensation hydrogenation reaction in presence of a catalyst, wherein the compound A is selected from one or more of 3,5,5-trimethyl-2-cyclohexen-1-one, 3,3,5-trimethylcyclohexanone, and 3,3,5-trimethylcyclohexanol.

In one or more embodiments, the method comprises continuously conducting the condensation hydrogenation reaction using a fixed bed reactor, such as a trickle bed reactor.

In one or more embodiments, the catalyst is selected from one or more of a Pd/C catalyst, a Pt/C catalyst, a Pt-S/C catalyst, and a Pb/C catalyst.

In one or more embodiments, a Pd content of the Pd/C catalyst is 0.1wt%~0.5wt%, a Pt content of the Pt/C catalyst and the Pt-S/C catalyst is 0.5wt%~3wt%, and a Pb content of the Pb/C catalyst is 1wt%~5wt%.

In one or more embodiments, a reaction temperature is 100°C~140°C.

In one or more embodiments, a reaction pressure is 1MPa~3MPa.

In one or more embodiments, an intake flow rate of hydrogen is 3 0mL/min~100mL/min.

In one or more embodiments, a molar ratio of the compound A to 4-aminodiphenylamine is 2:1-5:1.

In one or more embodiments, a total feed rate of the compound A and 4-aminodiphenylamine is 0.2mL/min~1.5mL/min.

The present invention also provides a rubber composition, wherein the rubber composition comprises N-(3,5,5-trimethylcyclohexyl)-N'-phenyl p-phenylenediamine.

The present invention also provides a rubber article, wherein the rubber article comprises the rubber composition as described in any one of the embodiments herein; preferably, the rubber article is a tire.

The present invention also provides a rubber antidegradant, wherein the rubber antidegradant comprises N-(3,5,5-trimethylcyclohexyl)-N'-phenyl p-phenylenediamine.

The present invention also provides a use of N-(3,5,5-trimethylcyclohexyl)-N'-phenyl p-phenylenediamine in increasing vulcanization speed of rubber compositions, shortening scorch time of rubber compositions, and/or improving thermal oxidative aging resistance of rubber compositions.

### BRIEF DESCRIPTION OF THE FIGURES

Figure 1 shows a comparative graph of the flexing resistance of the vulcanized films in Example 1 and Comparative Example.
Figure 2 shows a comparative graph of the tensile fatigue resistance of the vulcanized films in Example 1 and Comparative Example.

### DETAILED DESCRIPTION

In order to enable those skilled in the art to understand the features and effects of the present invention, the following is a general description and definition of terms and words mentioned in the specification and claims. Unless otherwise specified, all technical and scientific terms used herein are intended to be the ordinary meaning of the knowledge of the present disclosure by those skilled in the art, and in case of a conflict, the definition of this specification shall prevail.

The theories or mechanisms described and disclosed herein, whether right or wrong, should not limit the scope of the present disclosure in any way, that is to say the present disclosure can be practiced without limitation to any particular theory or mechanism.

In the present invention, all features, such as numerical values, quantities, amounts and concentrations, which are defined by numerical ranges or percentage ranges, are only for the sake of simplicity and convenience. Accordingly, the recitation of numerical ranges or percentage ranges shall be construed as covering and specifically disclosing all possible sub-ranges and individual values (including integers and fractions) in the range.

Herein, the sum of the percentages of each component in the composition is 100%.

Herein, for the sake of brevity of description, all possible combinations of various technical features in the various embodiments or Examples are not described. Therefore, as long as there is no contradiction in the combination of these technical features, the various technical features in the various embodiments or Examples can be combined in any combination, and all possible combinations should be considered to be within the scope of this specification.

The present invention finds that N-(3,5,5-trimethylcyclohexyl)-N'-phenyl p-phenylenediamine can be used as a rubber antidegradant. Compared with antidegradant 6PPD, N-(3,5,5-trimethylcyclohexyl)-N'-phenyl p-phenylenediamine can increase the vulcanization speed of a rubber material, shorten scorching time, improve thermal oxidative aging resistance of the rubber, and at the same time, keep good original physical properties, flexing resistance and dynamic fatigue resistance. In addition, compared with antidegradant 4010 (N-cyclohexyl-N'-phenyl p-phenylenediamine), N-(3,5,5-trimethylcyclohexyl)-N'-phenyl p-phenylenediamine has greater solubility, better compatibility, stronger ozone resistance, easy dispersion, less migration, and less prone to frost spraying in rubber, making it particularly suitable for natural rubber and synthetic rubber.

N-(3,5,5-trimethylcyclohexyl)-N'-phenyl p-phenylenediamine can be synthesized by condensation hydrogenation of a compound A, 4-amino diphenylamine and hydrogen in the presence of a catalyst. In the present invention, the compound A is selected from one or more of 3,5,5-trimethyl-2-cyclohexen-1-one, 3,3,5-trimethylcyclohexanone, and 3,3,5-trimethylcyclohexanol.

In some embodiments, the compound A and 4-aminodiphenylamine are used as raw materials to carry out continuous condensation hydrogenation reaction in a fixed bed reactor in the presence of a catalyst to obtain N-(3,5,5-trimethylcyclohexyl)-N'-phenyl p-phenylenediamine. Compared with kettle type intermittent reactions, the continuous production of N-(3,5,5-trimethylcyclohexyl)-N'-phenyl p-phenylenediamine using the fixed bed is more suitable for industrial application. The available fixed bed reactor includes but is not limited to a trickle bed reactor.

The available catalyst is selected from one or more of a Pd/C catalyst, a Pt/C catalyst, a Pt-S/C catalyst, and a Pb/C catalyst. The Pd content of the Pd/C catalyst can be 0.1wt%~0.5wt%, such as 0.2wt%, 0.25wt%, 0.3wt%, 0.35wt%, and 0.4wt%. The Pt content of the Pt/C catalyst and the Pt-S/C catalyst can be 0.5wt%~3wt%, such as 0.5wt%, 1wt%, 1.5wt%, 2wt%, and 3wt%. The Pb content of the Pb/C catalyst can be 1wt%~5wt%, such as 2wt%, 3wt%, and 4wt%.The Pd/C catalyst, the Pt/C catalyst, the Pt-S/C catalyst and the Pb/C catalyst suitable for the present invention are commercially available.

In the preparation of N-(3,5,5-trimethylcyclohexyl)-N'-phenyl p-phenylenediamine, the reaction temperature can be 100°C~140°C, such as 115 °C, 120 °C, 130 °C, and 140 °C. The reaction pressure can be 1MPa~3MPa, such as 1MPa, 1.2MPa, 1.5MPa, 2MPa, 2.5MPa, and 3MPa. The intake flow rate of hydrogen can be 30mL/min~100mL/min, such as 30mL/min, 60mL/min, 80mL/min, 90mL/min, and 100mL/min. The molar ratio of the compound A to 4-amino diphenylamine can be 2:1-5:1, such as 2.5:1, 3:1, 4:1. The total feed rate of the compound A and 4-aminodiphenylamine can be 0.2mL/min~1.5mL/min, such as 0.2mL/min, 0.25mL/min, 0.42mL/min, 1.5mL/min. The reaction time can be when the liquid space velocity of the reaction solution reaches 2h⁻¹-4h⁻¹, such as 2h⁻¹, 3h⁻¹, 3.3h⁻¹, 3.5h⁻¹, 4h⁻¹.

The method for preparing N-(3,5,5-trimethylcyclohexyl)-N'-phenyl p-phenylenediamine provided by the present invention can achieve the following beneficial effects:
(1) The preparation method of the present invention improves the utilization rate of raw materials, has low production energy consumption and high product content, and overcomes the defects of high reaction pressure, high reaction temperature, easy caking in the process of product storage and transportation, and the like of the existing fixed bed process.
(2) The Pt/C catalyst used in the preparation method of the present invention has a low loading capacity and can react at low temperature and low pressure, avoiding the need for high temperature and high pressure in kettle type intermittent reactions; the method of the present invention reduces the fixed assets investment of the device on the one hand, and increases the safety on the other hand; the method of the present invention avoids the residue of copper-based catalysts in the product due to breakage, and the product has a wider range of applications; the product of the present invention has a high initial melting point and is not prone to agglomerate; specifically, the side reaction of hydrogenation of ketones to generate corresponding alcohols is effectively suppressed.
(3) The Pt-S/C catalyst used in the preparation method of the present invention has high activity, good selectivity, and can be continuously used.
(4) The preparation method of the present invention reduces the ketone-amine ratio of the raw material, reduces the energy consumption due to the reduction of the ketone-amine ratio, simultaneously reduces the load of post-treatment, and obviously reduces the production cost.

The present invention also provides a rubber composition, wherein the rubber composition comprises N-(3,5,5-trimethylcyclohexyl)-N'-phenyl p-phenylenediamine as an antidegradant.

The raw materials of the rubber composition typically comprise a diene elastomer, a reinforcing filler, an antidegradant, and a crosslinker. In the present invention, the rubber composition includes unvulcanized rubber and vulcanized rubber. Vulcanized rubber may be prepared by vulcanizing (curing) unvulcanized rubber.

The raw materials of the rubber composition of the present invention comprise a diene elastomer, a reinforcing filler, an antidegradant, and a crosslinker, wherein, based on 100 parts by weight of the diene elastomer, the content of the reinforcing filler can be 30-70 parts by weight, the content of the antidegradant can be 0.1-8 parts by weight, and the content of the crosslinker can be 0.5-3 parts by weight, and the antidegradant comprises N-(3,5,5-trimethylcyclohexyl)-N'-phenyl p-phenylenediamine. In the present invention, unless otherwise specified, the parts by weight of the raw materials of the rubber composition are based on 100 parts by weight of the diene elastomer.

In the present invention, a diene elastomer refers to an elastomer with its monomers comprising dienes (such as butadiene and isoprene). The diene elastomer suitable for the present invention may be various diene elastomers known in the art, including, but not limited to, one or more selected from natural rubber (NR), butadiene rubber (BR), isoprene rubber, styrene-butadiene rubber (SBR), chloroprene rubber (CR), nitrile rubber (NBR), isoprene/butadiene copolymer, isoprene/styrene copolymer, and isoprene/butadiene/styrene copolymer. In some embodiments, in the raw materials of the rubber composition of the present invention, the diene elastomer comprises a natural rubber and a butadiene rubber, or consists of a natural rubber and a butadiene rubber. The mass ratio of the natural rubber to the butadiene rubber may be from 1:9 to 9:1, from 2:8 to 8:2, from 3:7 to 7:3, from 4:6 to 6:4, from 4.5:5.5 to 5.5:4.5, or 1: 1. Examples of the natural rubber comprise SCR5. Examples of the butadiene rubber comprise BR9000.

The rubber composition of the present invention typically comprises 0.1-8 parts by weight, preferably 1-5 parts by weight, such as 2 parts by weight, 2.5 parts by weight, 3 parts by weight, 3.5 parts by weight, and 4 parts by weight of an antidegradant. The characteristic of the rubber composition of the present invention is that the antidegradant contained in the rubber composition comprises N-(3,5,5-trimethylcyclohexyl)-N'-phenyl p-phenylenediamine. The rubber composition of the present invention may comprise 0.1-8 parts by weight, preferably 1-5 parts by weight, such as 2 parts by weight, 2.5 parts by weight, 3 parts by weight, 3.5 parts by weight, and 4 parts by weight of N-(3,5,5-trimethylcyclohexyl)-N'-phenyl p-phenylenediamine. The rubber composition of the present invention may also comprise other antidegradant, such as antidegradant TMQ (2,2,4-trimethyl-1,2-dihydroquinoline polymer). In some embodiments, the antidegradant contained in the rubber composition of the present invention comprises N-(3,5,5-trimethylcyclohexyl)-N'-phenyl p-phenylenediamine and antidegradant TMQ, or consists of N-(3,5,5-trimethylcyclohexyl)-N'-phenyl p-phenylenediamine and antidegradant TMQ. The preferred antidegradant TMQ for the present invention is antidegradant S-TMQ, which is an antidegradant TMQ with the total amount of 2,2,4-trimethyl-1,2-dihydroquinoline dimers and trimers ≥70%. The combination of N-(3,5,5-trimethylcyclohexyl)-N'-phenyl p-phenylenediamine and antidegradant TMQ can increase the vulcanization speed of the rubber material, shorten scorching time, improve thermal oxidative aging resistance of the rubber, and at the same time, keep good original physical properties, flexing resistance and dynamic fatigue resistance. When the antidegradant contained in the rubber composition is a combination of N-(3,5,5-trimethylcyclohexyl)-N'-phenyl p-phenylenediamine and other antidegradant, the mass ratio of N-(3,5,5-trimethylcyclohexyl)-N'-phenyl p-phenylenediamine to other antidegradant (such as antidegradant TMQ) can be from 1:5 to 5:1, preferably from 1:1 to 5:1, such as 2:1, 5:2, and 3:1.

A reinforcing filler suitable for the present invention may be a reinforcing filler conventionally used in the rubber composition, including, but not limited to, one or more selected from carbon black, titanium oxide, magnesium oxide, calcium carbonate, magnesium carbonate, aluminum hydroxide, magnesium hydroxide, clay, and talc. In some embodiments, in the rubber composition of the present invention, the reinforcing filler is carbon black. The raw materials of the rubber composition typically comprise 30-70 parts by weight, preferably 40-60 parts by weight, and more preferably 45-55 parts by weight of a reinforced filler. In some embodiments, the raw materials of the rubber composition of the present invention comprise 30-70 parts by weight, preferably 40-60 parts by weight, more preferably 45-55 parts by weight, such as 50±2 parts by weight of carbon black.

A crosslinker may be sulfur. The raw materials of the rubber composition usually comprise 0.5-3 parts by weight, preferably 1-3 parts by weight, and more preferably 1-2 parts by weight of a crosslinker. In some embodiments, the raw materials of the rubber composition of the present invention comprise 0.5-3 parts by weight, preferably 1-3 parts by weight, more preferably 1-2 parts by weight, such as 1.5 ± 0.2 parts by weight, 1.5 ± 0.1 parts by weight of sulfur.

The raw materials of the rubber composition of the present invention may also comprise other components commonly used in the rubber composition, including but not limited to one or more of aids and promoters. The respective amounts of aids and promoters may be conventional amounts known in the art.

Aids may include softeners used to improve properties such as processability. The softeners may include petroleum softeners (operating oils) such as naphthenic oils, aromatic oils, processing oils, lubricating oils, paraffins, liquid paraffins, petroleum asphalt, and vaseline, etc., and may also include fatty oil softeners such as stearic acid, castor oil, flaxseed oil, rapeseed oil, coconut oil, waxes (such as beeswax, carnauba wax and lanolin), tall oil, linoleic acid, palmitic acid, and lauric acid, etc. Aids may also include activators, such as zinc oxide, which can accelerate the vulcanization rate and improve thermal conductivity, wear resistance, and tear resistance of the rubber. Aids may also include a protective wax, which plays a role in physical anti-aging and prolonging service life. Typically, aids are used in an amount of a total of 2-20 parts by weight per 100 parts by weight of the diene elastomer. In some embodiments, the raw materials of the rubber composition of the present invention comprise a petroleum softener, such as naphthenic oil. The raw materials of the rubber composition of the present invention can comprise 0-20 parts by weight, preferably 1-10 parts by weight, more preferably 2-8 parts by weight, such as 5 ± 2 parts by weight, or 5 ± 1 parts by weight of a petroleum softener, such as naphthenic oil. In some embodiments, the raw materials of the rubber composition of the present invention comprise a fatty oil softener, such as stearic acid. The raw materials of the rubber composition of the present invention can comprise 0-5 parts by weight, preferably 0.5-4 parts by weight, more preferably 1-3 parts by weight, such as 2 ± 0.5 parts by weight, or 2 ± 0.2 parts by weight of a fatty oil softener, such as stearic acid. In some embodiments, the raw materials of the rubber composition of the present invention comprise an activator, such as zinc oxide. The raw materials of the rubber composition of the present invention can comprise 0-10 parts by weight, preferably 2-8 parts by weight, more preferably 3-7 parts by weight, such as 5 ± 1 parts by weight of an activator, such as zinc oxide. In some embodiments, the raw materials of the rubber composition of the present invention comprise a protective wax. The raw materials of the rubber composition of the present invention can comprise 0-5 parts by weight, preferably 1-3 parts by weight, such as 1.5 ± 0.5 parts by weight of a protective wax. In some embodiments, the raw materials of the rubber composition of the present invention comprise a petroleum softener, a fatty oil softener, an activator, and a protective wax, wherein the respective amounts of the petroleum softener, the fatty oil softener, the activator, and the protective wax can be as described earlier.

Promoters are usually vulcanization accelerators, which can be one or more of sulfonamide vulcanization accelerators, thiazole vulcanization accelerators, thiuram vulcanization accelerators, thiourea vulcanization accelerators, guanidine vulcanization accelerators, dithiocarbamate vulcanization accelerators, aldehyde amine vulcanization accelerators, aldehyde ammonia vulcanization accelerators, imidazoline vulcanization accelerators, or xanthic acid vulcanization accelerators. For example, the promoter may be promoter NS (N-tert-butyl-2-benzothiazole sulfenamide). In some embodiments, the raw materials of the rubber composition of the present invention comprises a promoter, such as promoter NS. The raw materials of the rubber composition of the present invention can comprise 0-1.5 parts by weight, preferably 0.5-1.5 parts by weight, more preferably 0.5-1.2 parts by weight, for example, 0.8 ± 0.2 parts by weight, 0.8 ± 0.1 parts by weight of a promoter, such as promoter NS.

In addition, when necessary, a plasticizer may be used in the rubber composition, for examples, DMP (dimethyl phthalate), DEP (diethyl phthalate), DBP (dibutyl phthalate), DHP (diheptyl phthalate), DOP (dioctyl phthalate), DINP (diisononyl phthalate), DIDP (diisodecyl phthalate), BBP (butyl benzyl phthalate), DWP (dilauryl phthalate), and DCHP (dicyclohexyl phthalate). The amount of the plasticizer may be a conventional amount in the art.

In some preferred embodiments, the raw materials of the rubber composition of the present invention comprise: 100 parts by weight of a diene elastomer, 40-60 parts by weight, preferably 45-55 parts by weight of a reinforcing filler, 1-3 parts by weight, preferably 1-2 parts by weight of sulfur, 2-5 parts by weight, preferably 2.5-4.5 parts by weight of an antidegradant, 2-8 parts by weight, preferably 3-7 parts by weight of an activator, 0.5-4 parts by weight, preferably 1-3 parts by weight of a fatty oil softener, 1-10 parts by weight, preferably 2-8 parts by weight of a petroleum softener, 0.5-5 parts by weight, preferably 1-3 parts by weight of a protective wax, and 0.5-1.5 parts by weight, preferably 0.5-1.2 parts by weight of a promoter; wherein, the diene elastomer preferably comprise natural rubber and butadiene rubber with a mass ratio of from 4:6 to 6:4, preferably from 4.5:5.5 to 5.5:4.5; the reinforcing filler is preferably carbon black; the antidegradant comprises N-(3,5,5-trimethylcyclohexyl)-N'-phenyl p-phenylenediamine, and optionally may further comprise antidegradant TMQ; the activator is preferably zinc oxide; the fatty oil softener is preferably stearic acid; the ppetroleum softener is preferably naphthenic oil; the promoter is preferably promoter NS.

The unvulcanized rubber of the present invention can be prepared by a conventional rubber mixing method, such as a two-stage mixing method as follows: in the first stage, diene elastomers, reinforcing fillers, aids, and antidegradants are mixed in an internal mixer, and the rubber discharge temperature is 110°C or higher; in the second stage, the rubber obtained in the first stage with crosslinkers and promoters are mixed in an open mill. In general, a diene elastomer is added into a thermo-mechanical mixer (such as an internal mixer) at first. After kneading for a certain period of time, a reinforcing filler, an aid, and an antidegradant are added, and the kneading is continued until the mixture is uniformly mixed, wherein the reinforcing filler, the aid, and the antidegradant can be added in batches. The temperature during the kneading is controlled to be between 110°C and 190°C, such as between 130°C and 140°C. The mixture is then cooled to 100 °C or lower. A crosslinker and a promoter are added to the mixture and a second kneading is performed during which the temperature is controlled to 110°C or lower, e.g. 50±5 °C, and the unvulcanized rubber is obtained.

The unvulcanized rubber of the present invention may be vulcanized by a conventional vulcanization method to obtain a vulcanized rubber. The vulcanization temperature is usually 130°C-200°C, such as 140°C-150°C, or 145±2°C. The vulcanization time depends on the vulcanization temperature, vulcanization system, and vulcanization kinetics, and is usually 15-60 minutes, such as 20-30 minutes, or 25±2 minutes. Before vulcanization, conventional tabletting can be performed on the kneaded unvulcanized rubber.

In some embodiments, the rubber composition of the present invention is prepared by the following method:
(1) mixing diene elastomers, reinforcing fillers, aids, and antidegradants uniformly with a thermo-mechanical mixer (such as an internal mixer), preferably with a rubber discharge temperature of 110°C or higher, such as 135 ± 5 °C;
(2) mixing the rubber material obtained in step (1), vulcanizing agents, and promoters uniformly with a thermo-mechanical mixer (such as an open mill), preferably with a tablet discharge temperature of 110°C or lower, for example, 50 ± 5°C, to obtain an unvulcanized rubber.
   In some embodiments, the method for preparing the rubber composition of the present invention further comprises:
(3) after tabletting the unvulcanized rubber optionally, vulcanizing the unvulcanized rubber to obtain a vulcanized rubber. Preferably, the vulcanization temperature is 130°C-200°C, such as 140-150 °C, and preferably, the vulcanization time is 15-60 minutes, such as 20-30 minutes.

The use of the rubber composition of the present invention in rubber articles, especially rubber tires, can increase the vulcanization speed of a rubber material, shorten scorching time, improve thermal oxidative aging resistance of the rubber, and at the same time, keep good original physical properties, flexing resistance and dynamic fatigue resistance.

Therefore, the present invention also provides a rubber article comprising the rubber composition described herein. The rubber article may be a tire, a rubber shoe, a sealing strip, a sound insulation panel, or a shock absorbing pad. In some embodiments, the rubber product is a tire, such as a tread, a belt ply, or a sidewall of a tire. As a belt ply of a tire, the rubber article may further comprise a reinforcing material conventionally used in the art in addition to the rubber composition of the present invention.

The present invention will be described below in the form of specific examples. It should be understood that these examples are merely illustrative and are not intended to limit the scope of the present description. Unless otherwise specified, the methods, reagents and materials used in the following examples and comparative examples are conventional in the art. The raw materials used in the examples and comparative examples are commercially available.

### Preparation Example 1

A certain amount of Pd/C catalyst (Shanghai Xunkai Technology N3001 catalyst, the content of Pd is 0.35%) is filled in a trickle bed reactor, so that a bed filling height of the catalyst is 40 cm. A hydrogen cylinder is used to replace the air 6 times. The reaction device is heated until the temperature reaches 100°C, and hydrogen is introduced and tail gas is discharged. The hydrogen pressure is 1.5MPa and the flow rate of the tail gas is 30 mL/min. A mixture of 3,5,5-trimethyl-2-cyclohexen-1-one and 4-ADPA (with a molar ratio of 4:1) is pumped into the system with a constant flow pump at a rate of 0.25 mL/min. The system pressure is 1.5MPa and the reaction temperature is 115°C. After the reaction solution reaches a liquid space velocity of 3h⁻¹, samples are taken and analyzed by GC. The conversion rate of 4-ADPA is 99.14%. The selectivity of N-(3,5,5-trimethylcyclohexyl)-N'-phenyl p-phenylenediamine is 98.52%.

### Preparation Example 2

A certain amount of Pt/C catalyst (Shanghai Xunkai Technology N4001 catalyst, the content of Pt is 0.5%) is filled in a trickle bed reactor, so that a bed filling height of the catalyst is 40 cm. A hydrogen cylinder is used to replace the air 6 times. The reaction device is heated until the temperature reaches 90 °C , and hydrogen is introduced and tail gas is discharged. The hydrogen pressure is 1.0MPa and the flow rate of the tail gas is 60 mL/min. A mixture of 3,3,5-trimethyl-cyclohexanone and 4-ADPA (with a molar ratio of 3:1) is pumped into the system with a constant flow pump at a rate of 0.20 mL/min. The system pressure is 1.0MPa and the reaction temperature is 120°C. After the reaction solution reaches a liquid space velocity of 4h⁻¹, samples are taken and analyzed by GC. The conversion rate of 4-ADPA is 99.95%. The selectivity of N-(3,5,5-trimethylcyclohexyl)-N'-phenyl p-phenylenediamine is 98.82%.

### Preparation Example 3

A certain amount of Pt-S/C catalyst (Shanghai Xunkai Technology N4002 catalyst, the content of Pt is 1.5%) is filled in a trickle bed reactor, so that a bed filling height of the catalyst is 40 cm. A hydrogen cylinder is used to replace the air 6 times. The reaction device is heated until the temperature reaches 95°C, and hydrogen is introduced and tail gas is discharged. The hydrogen pressure is 1.2MPa and the flow rate of the tail gas is 100 mL/min. A mixture of 3,5,5-trimethyl-2-cyclohexen-1-one and 4-ADPA (with a molar ratio of 2.5:1) is pumped into the system with a constant flow pump at a rate of 0.42 mL/min. The system pressure is 1.2MPa and the reaction temperature is 130°C. After the reaction solution reaches a liquid space velocity of 2h⁻¹, samples are taken and analyzed by GC. The conversion rate of 4-ADPA is 99.2%. The selectivity of N-(3,5,5-trimethylcyclohexyl)-N'-phenyl p-phenylenediamine is 98.02%.

### Preparation Example 4

A certain amount of Pt/C catalyst (Shanghai Xunkai Technology N4003 catalyst, the content of Pt is 3%) is filled in a trickle bed reactor, so that a bed filling height of the catalyst is 40 cm. A hydrogen cylinder is used to replace the air 6 times. The reaction device is heated until the temperature reaches 100°C, and hydrogen is introduced and tail gas is discharged. The hydrogen pressure is 2.5MPa and the flow rate of the tail gas is 60 mL/min. A mixture of 3,5,5-trimethyl-2-cyclohexen-1-one and 4-ADPA (with a molar ratio of 4:1) is pumped into the system with a constant flow pump at a rate of 0.25 mL/min. The system pressure is 2.5MPa and the reaction temperature is 120°C. After the reaction solution reaches a liquid space velocity of 3.3h⁻¹, samples are taken and analyzed by GC. The conversion rate of 4-ADPA is 99.5%. The selectivity of N-(3,5,5-trimethylcyclohexyl)-N'-phenyl p-phenylenediamine is 70.6%.

### Preparation Example 5

A certain amount of Pt-S/C catalyst (Shanghai Xunkai Technology N4003s catalyst, the content of Pt is 3%) is filled in a trickle bed reactor, so that a bed filling height of the catalyst is 40 cm. A hydrogen cylinder is used to replace the air 6 times. The reaction device is heated until the temperature reaches 140°C, and hydrogen is introduced and tail gas is discharged. The hydrogen pressure is 3.0MPa and the flow rate of the tail gas is 90 mL/min. A mixture of 3,5,5-trimethyl-2-cyclohexen-1-one and 4-ADPA (with a molar ratio of 3:1) is pumped into the system with a constant flow pump at a rate of 1.5 mL/min. The system pressure is 3.0MPa and the reaction temperature is 140°C. After the reaction solution reaches a liquid space velocity of 3.3h⁻¹, samples are taken and analyzed by GC. The conversion rate of 4-ADPA is 99.9%. The selectivity of N-(3,5,5-trimethylcyclohexyl)-N'-phenyl p-phenylenediamine is 98%.

### Preparation Example 6

A certain amount of Pb/C catalyst (Shanghai Xunkai Technology N3002 catalyst, the content of Pb is 3%) is filled in a trickle bed reactor, so that a bed filling height of the catalyst is 40 cm. A hydrogen cylinder is used to replace the air 6 times. The reaction device is heated until the temperature reaches 140°C , and hydrogen is introduced and tail gas is discharged. The hydrogen pressure is 3.0MPa and the flow rate of the tail gas is 90 mL/min. A mixture of 3,3,5-trimethyl-2-cyclohexanol and 4-ADPA (with a molar ratio of 3:1) is pumped into the system with a constant flow pump at a rate of 1.5 mL/min. The system pressure is 3.0MPa and the reaction temperature is 140°C. After the reaction solution reaches a liquid space velocity of 3.3h⁻¹, samples are taken and analyzed by GC. The conversion rate of 4-ADPA is 98.5%. The selectivity of N-(3,5,5-trimethylcyclohexyl)-N'-phenyl p-phenylenediamine is 98.2%.

### Example and Comparative Example: Preparation of Vulcanized Films

The rubber compositions of Example 1 and Comparative Example are prepared by a two-stage mixing process according to the formulation described in Table 1, comprising the following steps:
1. first stage of mixing in an internal mixer: a natural rubber (NR), a synthetic rubber BR9000, carbon black N550, zinc oxide, stearic acid (SA), protective wax, operating oil, and antidegradants are added into an internal mixer, the mixture is mixed uniformly, and the rubber discharge temperature is 135 °C ;
2. second stage of mixing in an open mill: the rubber material obtained in the first stage, sulfur (S), and promoter NS are added into an open mill, the mixture is mixed uniformly, and the film discharge temperature is 50 °C;
3. vulcanization: the rubber material obtained in the second stage is calendered into an unvulcanized film with a thickness of 2mm, and vulcanized at 145 °C for 25 minutes to obtain a vulcanized rubber film.

The sources of the raw materials used in the Examples and Comparative Example are as follows:
NR: SCR5, Xishuangbanna Sinochem Rubber Co., Ltd.;
BR900: synthetic rubber BR9000, Nanjing Yangzi Petrochemical Rubber Co., Ltd.;
N550: carbon black N550, Cabot Corporation;
Zinc oxide: general reagent zinc oxide (AR), Shanghai Titan Scientific Co., Ltd.
Stearic acid: general reagent stearic acid (AR), Shanghai Titan Scientific Co., Ltd.
Protective wax: H2830, Shandong Yanggu Huatai;
Naphthenic oil: Kunlun Naphthenic Oils KL4006, Xinjiang Kelian;
Antidegradant 6PPD: Sennics Co., Ltd;
Antidegradant MZG6: N-(3,5,5-trimethylcyclohexyl)-N'-phenyl p-phenylenediamine;
Antidegradant S-TMQ: Sennics Co., Ltd.
Promoter NS: Sennics Co., Ltd;
S: sublimed sulfur (AR), Sinopharm Chemical Reagent Co., Ltd.

**Table 1: Formulation of the rubber composition (unit: parts by mass)**

| | Comparative Example | Example 1 |
|---|---|---|
| NR | 50 | 50 |
| BR9000 | 50 | 50 |
| N550 | 50 | 50 |
| ZnO | 5 | 5 |
| SA | 2 | 2 |
| Protective wax | 1.5 | 1.5 |
| Naphthenic oil | 5 | 5 |
| Antidegradant 6PPD | 2.5 | |
| Antidegradant MZG6 | | 2.5 |
| Antidegradant S-TMQ | 1.0 | 1.0 |
| Promoter NS | 0.8 | 0.8 |
| S | 1.5 | 1.5 |
| Total | 169.3 | 169.3 |

### Test Example 1: Vulcanization Speed and Degree

According to GB/T16584-1996 (Rubber-Measurement of vulcanization characteristics with rotorless curemeters), the vulcanization speed and degree of the rubber material are measured with a rheometer (145 °C * 60 min), and the results are shown in Table 2.

**Table 2: Vulcanization speed and degree of the rubber material**

| | Comparative Example | Example 1 |
|---|---|---|
| t₁₀/min | 8.02 | 7.34 |
| t₉₀/min | 16.58 | 14.95 |
| MH-ML/dNm | 12.5 | 12.3 |

In Table 2, "MH-ML" represents vulcanization degree, which directly affects the physical properties of the rubber material. It can be seen from Table 2 that the vulcanization speed of the rubber material of Example 1 comprising N-(3,5,5-trimethylcyclohexyl)-N'-phenyl p-phenylenediamine is increased by about 10% compared with that of the rubber material comprising antidegradant 6PPD, and the vulcanization degrees are at the same level.

### Test Example 2: Scorching Time

According to GB/T1233-2008 (Rubber unvulcanized-Determinations of pre-vulcanization characteristic), scorching time of the rubber material is measured with a Mooney meter (120 °C, ts), and the results are shown in Table 3.

**Table 3: Scorching time of the rubber material**

| | Comparative Example | Example 1 |
|---|---|---|
| t₅/min | 36.66 | 32.78 |
| t₃₅/min | 40.71 | 35.69 |

It can be seen from Table 3 that scorching time of the rubber material of Example 1 comprising N-(3,5,5-trimethylcyclohexyl)-N'-phenyl p-phenylenediamine is shortened by about 10% compared with that of the rubber material comprising antidegradant 6PPD, and the effect on the Mooney of the rubber material is consistent.

### Test Example 3: Original Physical Properties and Thermal Oxidative Aging resistance

According to the following rubber testing standards, the original physical properties and thermal oxidative aging resistance of the vulcanized films in Example 1 and Comparative Example are tested, and the test results are shown in Table 4:
Elongation at break and tensile strength: GB/T 528-2009, Rubber, vulcanized or thermoplastic-Determination of tensile stress-strain properties;
Tear strength: GB/T 529-2008, Rubber, vulcanized or thermoplastic-Determination of tear strength;
Hardness: GB/T 531.1-2008, Rubber, vulcanized or thermoplastic-Determination of indentation hardness;
Thermal oxidative aging resistance: GB/T 3512-2001, Rubber, vulcanized or thermoplastic-Accelerated aging and heat resistance tests-Air-oven method.

**Table 4: Original physical properties and thermal oxidative aging resistance of the rubber material**

| | Comparative Example | Example 1 |
|---|---|---|
| Original physical properties | | |
| Tensile strength /MPa | 19.2 | 18.7 |
| Elongation at break /% | 572 | 574 |
| Tear strength /N·mm⁻¹ | 38.5 | 41.4 |
| Hardness /SHA | 57 | 57 |

| Thermal oxidative aging resistance (100°C *48 hour) | | |
|---|---|---|
| Retention of physical properties /% | 54 | 61 |
| Retention of physical properties /% | 39 | 43 |

It can be seen from Table 4 that the rubber material of Example 1 comprising N-(3,5,5-trimethylcyclohexyl)-N'-phenyl p-phenylenediamine has a relatively slightly higher tear strength and a slightly lower tensile strength than the rubber material comprising antidegradant 6PPD, and the elongation at break and hardness are basically the same. N-(3,5,5-trimethylcyclohexyl)-N'-phenyl p-phenylenediamine provides better thermal oxidative aging resistance than 6PPD, with a performance advantage of more than 5%.

### Test Example 4: Flexing Resistance

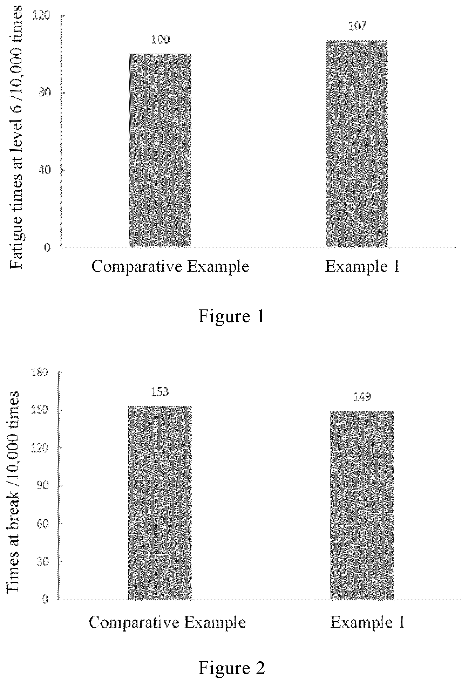

According to GB/T13934-2006 (Rubber, vulcanized or thermoplastic-Determination of flex cracking and crack growth (De mattia)), the flexing resistance performance of the vulcanized films in Example 1 and Comparative Example is tested, and the test results are shown in Table 5 and Figure 1.

### Test Example 5: Tensile Fatigue Resistance

According to GB/ T1688-2008 (Rubber, vulcanized-Determination of tensile fatigue), the tensile fatigue resistance of the vulcanized films in Example 1 and Comparative Example is tested, and the test results are shown in Table 5 and Figure 2.

**Table 5: Flexing resistance and tensile fatigue resistance of the rubber material**

| | Comparative Example | Example 1 |
|---|---|---|
| Fatigue times at level 6 /10,000 times | 100 | 107 |
| Times at break /10,000 times | 153 | 149 |

It can be seen from Table 5 that the flexing resistance and tensile fatigue resistance of the rubber material comprising 6PPD and the rubber material comprising N-(3,5,5-trimethylcyclohexyl)-N'-phenyl p-phenylenediamine are basically at the same level.

## Claims

1. A compound with the structure shown in the following formula:

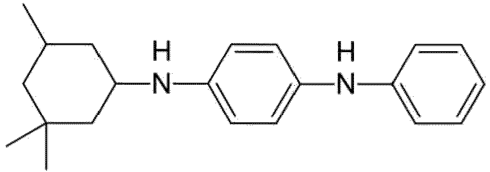

2. A method for preparing the compound of claim 1, wherein the method comprises reacting a compound A, 4-aminodiphenylamine and hydrogen in a condensation hydrogenation reaction in presence of a catalyst, wherein the compound A is selected from one or more of 3,5,5-trimethyl-2-cyclohexen-1-one, 3,3,5-trimethylcyclohexanone, and 3,3,5-trimethylcyclohexanol.

3. The method of claim 2, wherein the method comprises continuously conducting the condensation hydrogenation reaction using a fixed bed reactor, such as a trickle bed reactor.

4. The method of claim 2, wherein the catalyst is selected from one or more of a Pd/C catalyst, a Pt/C catalyst, a Pt-S/C catalyst, and a Pb/C catalyst.

5. The method of claim 4, wherein a Pd content of the Pd/C catalyst is 0.1wt%~0.5wt%, a Pt content of the Pt/C catalyst and the Pt-S/C catalyst is 0.5wt%~3wt%, and a Pb content of the Pb/C catalyst is 1wt%~5wt%.

6. The method of claim 2, wherein the method has one or more of the following features:
a reaction temperature is 100°C~140°C ;
a reaction pressure is 1MPa~3MPa;
an intake flow rate of hydrogen is 30mL/min~100mL/min;
a molar ratio of the compound A to 4-aminodiphenylamine is 2:1-5:1;
a total feed rate of the compound A and 4-aminodiphenylamine is 0.2mL/min~1.5mL/min.

7. A rubber composition, wherein the rubber composition comprises the compound of claim 1.

8. A rubber article, wherein the rubber article comprises the rubber composition of claim 7; preferably, the rubber article is a tire.

9. A rubber antidegradant, wherein the rubber antidegradant comprises the compound of claim 1.

10. A use of the compound of claim 1 in increasing vulcanization speed of rubber compositions, shortening scorch time of rubber compositions, and/or improving thermal oxidative aging resistance of rubber compositions.
